# EUROPÄISCHE PATENTSCHRIFT

(11) **EP 2 147 690 B1**
(45) Veröffentlichungstag und Bekanntmachung des Hinweises auf die Patenterteilung: **09.11.2011**
(21) Anmeldenummer: 08013404.2
(22) Anmeldetag: 25.07.2008
(51) Int. Cl.: A61M 1/02, A61M 5/44, A61F 7/00

(54) **Flüssigkeitswärmebeutel und Beutelwärmer**
Fluid warming bag and bag heater
Récipient de chauffage de liquide et dispositif de chauffage de récipient

(43) Veröffentlichungstag der Anmeldung: 27.01.2010
(73) Patentinhaber: WWT Technischer Gerätebau GmbH, 70190 Stuttgart (DE)
(72) Erfinder: Theilacker, Wolfgang, Dipl.-Ing., 70597 Stuttgart (DE); Theilacker, Matthias, 70563 Stuttgart (DE); Schmider, Klaus H., Dipl. Ing., 70180 Stuttgart (DE)
(74) Vertreter: Kohler Schmid Möbus

(56) Entgegenhaltungen:
- EP-A- 0 350 675
- WO-A-00/12155
- WO-A-2005/072666
- DE-A1-102004 026 446
- US-A- 5 733 263

## Beschreibung

Die Erfindung betrifft einen Flüssigkeitswärmebeutel für einen Beutelwärmer insbesondere zum Erwärmen von gekühltem Blut auf eine für eine Transfusion geeignete Temperatur. Ein derartiger Flüssigkeitswärmebeutel weist ein aus mindestens zwei an bevorzugt vier Begrenzungskanten miteinander verbundenen flexiblen Kunststofffolien gebildetes Durchflussvolumen mit einer flachen, im Wesentlichen trapezförmigen Grundform auf, das durch die Begrenzungskanten begrenzt wird. Am Durchflussvolumen sind eine Einlassleitungsöffnung und eine Auslassleitungsöffnung für eine zu wärmende Flüssigkeit vorgesehen.

Ein aus einem derartigen Blutwärmebeutel und einem Beutelwärmer bestehendes Blutwärmesystem wird zur Erwärmung von natürlichem Blut für Transfusionszwecke oder bei der Dialyse, aber auch für Transfusionen von Blutplasma oder anderen Flüssigkeiten verwendet.

Blut wird üblicherweise bei einer Temperatur von ca. 4°C aufbewahrt. Vor einer Verwendung muss dieses Blut daher auf Körpertemperatur von ca. 37°C aufgewärmt werden. Für Transfusionszwecke verwendetes Blut darf keine zu niedrige Temperatur und keine zu hohe Temperatur haben. Insbesondere in Notfällen muss das Erwärmen des Blutes sehr rasch durchgeführt werden, da kein erwärmtes Blut für eventuell auftretende Notfälle bereitgehalten werden kann. In Notfällen muss jedoch nicht nur das Erwärmen des Blutes rasch ermöglicht werden, auch die Handhabung des Blutwärmesystems muss einfach und rasch möglich sein. Die angegebenen Temperaturbedingungen müssen dabei unabhängig von der Durchflussgeschwindigkeit des Blutes durch das Blutwärmesystem eingehalten werden. Diese Durchflussgeschwindigkeit kann bei Druckinfusionen über 5 Liter pro Stunde betragen. Zur Vermeidung einer Überhitzung kann daher die Temperatur des Beutelwärmers nur geringfügig über der Körpertemperatur liegen, was zur Folge hat, dass, um eine ausreichende Wärmeübertragung zu erreichen, eine große Wärmeaustauschoberfläche vom Beutelwärmer zur Verfügung gestellt werden muss.

Beutelwärmer sind im Stand der Technik in verschiedenen Ausführungsformen bekannt. Unterschieden werden können Beutelwärmer zum Einlegen eines Flüssigkeitswärmebeutels, die eine ausklappbare, während des Aufwärmens eines eingelegten Flüssigkeitswärmebeutels geschlossene" Kammer aufweisen, in die der Flüssigkeitswärmebeutel eingelegt wird, und Beutelwärmer zum Einführen eines Flüssigkeitswärmebeutels in einen Spalt, der zwischen zwei mittels Befestigungsmitteln parallel zueinander fixierten Wärmetauscherplatten ausgebildet ist. Die Beutelwärmer mit einer aufklappbaren Kammer sind relativ umständlich zu handhaben und eignen sich daher nur bedingt für den Einsatz in Notfallsituationen.

Beim Erwärmen z.B. von Blut mittels eines Blutwärmesystems kann ein Ausgasen von im Blut gelösten Gasen und damit eine Gasblasenbildung im Flüssigkeitswärmebeutel auftreten. Diese Gasblasen dürfen nicht in den Körper des Patienten, dem das Blut zugeführt wird, gelangen, da sie eine Embolie auslösen könnten. Es sind daher Vorkehrungen zu treffen, um die Gasblasenzuführung zu vermeiden. Letzteres kann z.B. durch visuelle Beobachtung der Blutzuführung geschehen. Aus der US 6,572,641 ist ein gattungsgemäßer Flüssigkeitswärmebeutel bekannt, der zusätzlich zur Einlassleitungsöffnung und der Auslassleitungsöffnung eine in ein Gasaufnahmevolumen, das Teil des Durchflussvolumens ist, mündende Entgasungsöffnung aufweist, Diese Entgasungsöffnung ist im Bereich einer beim Flüssigkeitswärmen oben anzuordnenden oberen Begrenzungskante angeordnet und liegt oberhalb der Auslassleitungsöffnung. Dadurch können entstehende Gasblasen, die nach oben steigen, im Gasaufnahmevolumen aufgefangen und durch die Entgasungsöffnung abgeleitet werden. Bei diesem Flüssigkeitswärmebeutel reicht die Entgasungsöffnung relativ weit in das Gasaufnahmevolumen hinein, so dass der Flüssigkeitsspiegel der zu erwärmenden Flüssigkeit oberhalb der Entgasungsöffnung liegt. Letzteres kann zu einem Flüssigkeitsverlust führen, weshalb ein freies Ende eines an die Entgasungsöffnung angeschlossenen Schlauches gegen einen Flüssigkeitsdurchlass abdichtende Flüssigkeitsdurchflussverhinderungsmittel aufweist. Bei diesem Flüssigkeitswärmebeutel sind die Einlassleitungsöffnung, die Auslassleitungsöffnung und die Entgasungsöffnung jeweils an unterschiedlichen Begrenzungskanten, d.h. Seiten des Flüssigkeitswärmebeutels, angeordnet. Dadurch wird die Handhabung des Flüssigkeitswärmebeutels erschwert, da an die Öffnungen angeschlossene Schläuche ungeordnet aus dem zum Wärmen verwendeten Beutelwärmer herausführen.

Ein Blutwärmesystem mit einem besser handhabbaren gattungsgemäßen Flüssigkeitswärmebeutel ist in der DE 10 2004 026 446 A1 beschrieben. Bei diesem Flüssigkeitswärmebeutel sind die Einlassleitungsöffnung und die Auslassleitungsöffnung an einer beim Flüssigkeitswärmen oben anzuordnenden oberen Begrenzungskante angeordnet. Die obere Begrenzungskante verläuft derart schräg zu Ihren angrenzenden Begrenzungskanten, dass zwischen der oberen Begrenzungskante und einer daran angrenzenden Begrenzungskante ein spitzer Winkel eingeschlossen ist. Dieser spitze Winkel schließt einen oberen Durchflussvolumenbereich, ein in den die Auslassleitungsöffnung mündet. Weiter ist eine Flüssigkeitsflussführungsnaht vorgesehen, die von einer Verbindung der Kunststofffolien im Bereich des Durchflussvolumens gebildet wird und die obere Begrenzungskante in einen unteren Einiassteitungsöffnungsbereich und in einen oberen Auslassleitungsöffnungsbereich aufteilt. An die Öffnungen dieses Flüssigkeitswärmebeutels ragen geordnet nach oben aus einem verwendeten Beutelwärmer heraus. Jedoch weist dieser Flüssigkeitswärmebeutel keine Entgasungsöffnung auf.

Ein weiteres Ausführungsbeispiel eines Flüssigkeitswärmebeutels ist in der WO 00/12155 A1 beschrieben. Dieser Flüssigkeitswärmebeutel weist im Bereich eines spitzen Winkels an seiner oberen Begrenzungskante eine Entgasungsöffnung auf.

Der Erfindung liegt die Aufgabe zugrunde, einen Flüssigkeitswärmebeutel und ein Flüssigkeitswärmesystem bereitzustellen, welche die Nachteile des Standes der Technik vermeiden. Insbesondere soll unter Vermeidung von Verlusten der zu erwärmenden Flüssigkeit zuverlässig vermieden werden, dass beim Erwärmen entstehende Gasblasen in die Auslassleitungsöffnung gelangen.

Diese Aufgabe wird durch den Flüssigkeitswärmebeutel und das Flüssigkeitswärmesystem der Ansprüche gelöst. Die abhängigen Ansprüche stellen bevorzugte Ausführungsformen der Erfindung dar.

Der erfindungsgemäße Flüssigkeitswärmebeutel weist ein, aus mindestens zwei an bevorzugt vier Begrenzungskanten miteinander verbundenen flexiblen Kunststofffolien gebildetes Durchflussvolumen auf. Das Durchflussvolumen weist eine flache, im Wesentlichen trapezförmige Grundform auf, die durch die Begrenzungskanten begrenzt wird. Es sind eine Einlassleitungsöffnung und eine Auslassleitungsöffnung für eine zu wärmende Flüssigkeit vorgesehen, die an einer beim Flüssigkeitswärmen oben anzuordnenden oberen Begrenzungskante der Begrenzungskanten angeordnet sind. Die obere Begrenzungskante verläuft derart schräg zu ihren angrenzenden Begrenzungskanten, dass im Bereich eines von der oberen Begrenzungskante und einer der angrenzenden Begrenzungskanten eingeschlossenen spitzen Winkels vom Durchflussvolumen ein Gasaufnahmevolumen ausgebildet ist. Durch eine Flüssigkeitsflussführungsnaht, die von einer Verbindung der Kunststofffolien im Bereich des Durchflussvolumens gebildet wird, ist die obere Begrenzungskante in einen unteren Einlassleitungsöffnungsbereich und in einen oberen gasaufnahmevolumenseitigen Auslassleitungsöffnungsbereich aufgeteilt. Erfindungsgemäß ist an der oberen Begrenzungskante am Bereich des Gasaufnahmevolumens zwischen der Auslassleitungsöffnung und der an den Auslassleltungsöffnungsbereich angrenzenden Begrenzungskante eine Entgasungsöffnung angeordnet. Die Entgasungsöffnung liegt also bei für den Gebrauch vorgesehener Anordnung höher als die Auslassleitungsöffnung.

Der erfindungsgemäße Flüssigkeitswärmebeutel vereinigt die Vorteile der Blutbeutel der DE 10 2004 026 446 A1 und der US 6,572,641. Dadurch, dass die Entgasungsöffnung im Bereich des genannten spitzen Winkels der Grundform des Blutbeutels angeordnet ist, steht der Flüssigkeitsspiegel, d.h. der Flüssigkeitsstand, der zu erwärmenden Flüssigkeit im Gasaufnahmevolumen in einem relativ schmalen Volumenbereich an, so dass bereits bei einem geringfügigen Auftreten von Gasblasen ein für ein Vermeiden von Flüssigkeitsverlusten ausreichendes Gasvolumen, das leicht überwachbar ist, entsteht. Der Flüssigkeitsstand kann also leicht so geregelt werden, dass die Entgasungsöffnung immer oberhalb des Flüssigkeitsstandes liegt. Die Öffnungen des erfindungsgemäßen Flüssigkeitswärmebeutels sind alle an der oberen Begrenzungskante angeordnet, so dass daran angeschlossene Schläuche leicht geordnet geführt werden können. Letzteres ist insbesondere in Notfallsituationen, bei denen die Aufmerksamkeit von Helfern auf die Patienten fixiert ist, wichtig um Komplikationen durch z.B. abgeklemmte Schläuche zu vermeiden.

Zwischen der Auslassleitungsöffnung und der Entgasungsöffnung ist eine von der oberen Begrenzungskante abgehende und in das Durchflussvolumen hineinragende Trennnaht vorgesehen, die das Gasaufnahmevolumen vom Durchflussvolumen teilweise abtrennt.

Dadurch wird der Strom der zu erwärmenden Flüssigkeit im Flüssigkeitswärmebeutel auf eine definierte Bahn gelenkt, so dass eine gleichmäßige Erwärmung sicher gestellt ist.

Weiter ist an der Flüssigkeitsflussführungsnaht eine Gasblasenführungskante ausgebildet, die ausgehend von der Flüssigkeitsflussführungsnaht in das auslassleitungsöffnungsseitige Volumen des Durchflussvolumens mindestens so weit hineinragt, wie das freie Ende der Trennnaht von der Flüssigkeitsflussführungsnaht beabstandet ist. Dadurch werden Gasblasen, die im Bereich unterhalb der Auslassleitungsöffnung entstehen, auf die entgasungsöffnungseitige Seite der Trennnaht und damit in das Gasaufnahmevolumen umgeleitet. Letzteres führt zu einer besonders zuverlässigen Vermeidung des Ausleitens von Gasblasen durch die Auslassleitungsöffnung.

Wenn im auslassleitungsöffnungsseitigen Volumen des Durchflussvolumens mindestens eine Gasblasenaufstiegsbehinderungskante vorgesehen ist, die ein Gassammelvolumen ausbildet, kann eine Ansammlung von Gasblasen, d.h. Schaum, die in den Bereich der Auslassleitungsöffnung hineinreicht, zusätzlich erschwert werden. Zusätzlich kann in das Gassammelvolumen bei Bedarf ein das Gas von der Flüssigkeit die Trennung förderndes Material (poröser Körper und/oder Schaumstoff, usw.) eingelegt werden, damit eine sichere Trennung von Gas und Flüssigkeit gewährleistet wird.

Zur besonders einfachen Einführung des erfindungsgemäßen Flüssigkeitswärmebeutels in einen Beutelwärmer ist vorteilhaft eine Einführlasche an einer der oberen Begrenzungskante gegenüberliegenden unteren Begrenzungskante der Begrenzungskanten angeordnet. Die Einführlasche kann als Teil der Kunststofffolien ausgebildet sein, der über die untere Begrenzungskante herausragt. An dieser Lasche können auch Öffnungen und/oder Ösen vorgesehen sein, die ein Fixieren des Beutels in der korrekten Lage gewährleisten. In einer weiteren Ausführungsform kann bei eingesetztem Beutel In dem Beutelwärmer der Spalt mit den Flüssigkeitsein- und ausführungsleitungen und der Gasaustrittsleitung abgedeckt sein, damit keine Flüssigkeit in diesen Spalt gelangen kann. Diese Abdeckung kann Bestandteil des Beutels und/oder des Beutelwärmers sein.

Sehr kostengünstig herzustellende, als Einwegbeutel benutzbare, erfindungsgemäße Flüssigkeitswärmebeutel können dadurch hergestellt sein, dass das Durchflussvolumen dadurch ausgebildet ist, dass die Kunststofffolien an den Begrenzungskanten direkt miteinander verschweißt sind und die Flüssigkeitsflussführungsnaht als eine Schweißnaht zwischen den Kunststofffolien ausgebildet ist.

Wenn die Grundform des erfindungsgemäßen Flüssigkeltswärmebeutels lang gestreckt ausgebildet ist, wird der Durchflusswiderstand durch den Flüssigkeitswärmebeutel gering gehalten und Flüssigkeitsreste können leicht aus dem Flüssigkeitswärmebeutel ausgepresst werden. Dabei sind die sich gegenüberliegenden obere und untere Begrenzungskanten als Schmalseiten ausgebildet und die die obere und die untere Begrenzungskanten verbindenden Begrenzungskanten sind zueinander im Wesentlichen parallel verlaufend und als Langseiten ausgebildet. Die Flüssigkeitsflussführungsnaht verläuft dabei, beginnend an der oberen Begrenzungskante, zu den Langseiten im Wesentlichen parallel.

Ein erfindungsgemäßes Flüssigkeitswärmesystem umfasst einen Beutelwärmer und einen erfindungsgemäßen Flüssigkeitswärmebeutel.

Der Beutelwärmer ist zum Einführen eines erfindungsgemäßen Flüssigkeitswärmebeutels geeignet und weist Heizelemente, die zwei im Wesentlichen rechteckige Wärmetauscherplatten mit jeweils einer im Wesentlichen flächigen Wärmeaustauschoberfläche ausbilden, auf. Die Wärmetauscherplatten sind mit deren Wärmeaustauschoberfläche derart beabstandet sich gegenüberliegend angeordnet, dass zwischen den Wärmeaustauschoberflächen ein Spalt mit einer Spaltbreite zur Aufnahme des Flüssigkeitswärmebeutels ausgebildet ist. Erfindungsgemäß ist im Bereich der Wärmeaustauschoberflächen mindestens ein Flüssigkeitsstandsensor und/oder eine Beobachtungsöffnung zur Detektion eines Flüssigkeitsstandes in dem in den Beutelwärmer eingeführten Flüssigkeitswärmebeutel vorgesehen. Dadurch kann der Flüssigkeitsstand im eingelegten Flüssigkeitswärmebeutel zur Sicherstellung des Vermeidens von in die Auslassleitungsöffnung ausfließenden Gasblasen und zur Sicherstellung des Vermeidens von durch die Entgasungsöffnung ausfließender Flüssigkeit leicht beobachtet werden.

Wenn der Beutelwärmer an einer oberen Schmalseitenkante der Wärmetauscherplatten drei Nutansätze aufweist, eignet er sich besonders gut zur Verwendung mit einem erfindungsgemäßen Flüssigkeitswärmebeutel. Dabei sind die Nutansätze zu der Einlassleitungsöffnung und zu der Auslassleitungsöffnung und zu der Entgasungsöffnung des in den Beutelwärmer eingeführten Flüssigkeitswärmebeutels derart komplementär ausgeformt, dass eine an den Flüssigkeitswärmebeutel angeschlossene Einlaufleitung und eine an den Flüssigkeitswärmebeutel angeschlossene Auslassleitung und eine an den Flüssigkeitswärmebeutel angeschlossene Entgasungsleitung, während der Flüssigkeitswärmebeutel in den Beutelwärmer eingeführt ist, in den Nutansätzen formschlüssig gehalten sind.

Bevorzugt ist in mindestens einer der Wärmetauscherplatten im an die obere Schmalseitenkante der Wärmetauscherplatte angrenzenden Bereich eine Kavität, insbesondere eine Einfräsung, zur Formgebung des Gasaufnahmevolumens des Flüssigkeitswärmebeutels ausgebildet.

Besonders bevorzugt ist am Beutelwärmer ein Entgasungsventil zum Anschluss der Entgasungsöffnung des in den Beutelwärmer eingeführten Flüssigkeitswärmebeutels, bevorzugt über eine Schlauchleitung, vorgesehen. Mittels des Öffnens oder Schließens des Entgasungsventils kann der Flüssigkeitsstand in dem in den Beutelwärmer eingeführten Flüssigkeitswärmebeutel geregelt werden.

Sehr vorteilhaft sind der Flüssigkeitsstandsensor und das Entgasungsventil an eine Steuereinheit des Beutelwärmers angeschlossen. Dabei ist die Steuereinheit eingerichtet, bei einer durch den Flüssigkeitsstandsensor detektierten Überschreitung eines vorbestimmten Flüssigkeitsstandes im Gasaufnahmevolumen des Flüssigkeitswärmebeutels das Entgasungsventil zum Öffnen der Entgasungsöffnung zu steuern und/oder ein Abpumpen von im Gasaufnahmevolumen vorhandenem Gas mittels einer Pumpe zu steuern. Die Überwachung und Regelung des Flüssigkeitsstandes im Flüssigkeitswärmebeutel, d.h. im Gasaufnahmevolumen des Flüssigkeitswärmebeutels, kann so automatisch durchgeführt werden.

Bei dem Beutelwärmer können die Wärmetauscherplatten an einer ersten Längsseitenkante der Wärmetauscherplatten mit Befestigungsmitteln derart miteinander befestigt sein, dass die Wärmeaustauschoberflächen durch die Befestigungsmittel im Wesentlichen parallel zueinander fixiert sind, wobei der Spalt entlang einer zweiten Längsseitenkante und den Schmalseitenkanten der Wärmetauscherplatten offen ist. Dadurch ist das Einlegen eines erfindungsgemäßen Flüssigkeitswärmebeutels in den Beutelwärmer sehr schnell und einfach möglich. Dabei können die Befestigungsmittel derart federnd ausgebildet sein, dass eine in einem in den Beutelwärmer eingeführten Flüssigkeitswärmebeutel auftretende Druckveränderung zu einer Veränderung der Spaltbreite führt, wobei eine Druckerhöhung zu einer Aufweitung und eine Druckverminderung zu einer Einengung des Spaltes führt. Diese Spaltbreitenveränderung führt bereits selbsttätig zu einer Flüssigkeitsstandsregelung im Flüssigkeitswärmebeutel, da der Druck im Flüssigkeitswärmebeutel weitestgehend konstant gehalten wird.

An der Auslassleitung können auf deren weiteren Verlauf zum Patienten verschiedene bei Infusionen oder Transfusionen übliche Vorrichtungen an der Auslassleitung vorgesehen sein, z.B. eine Schlauchheizung oder eine Tropfkammer.

Die Erfindung wird nachfolgend anhand von Ausführungsbeispielen unter Bezugnahme auf die Zeichnungen näher erläutert.

Die Figuren 1a bis 1c zeigen verschiedene Ausführungsformen eines erfindungsgemäßen Flüssigkeitswärmebeutels.
Die Figuren 2a und 2b zeigen einen Beutelwärmer mit einem eingeführten erfindungsgemäßen Flüssigkeitswärmebeutel, wobei Figur 2b einen schematischen Querschnitt durch den Beutelwärmer mit einem eingeführten Flüssigkeitswärmebeutel zeigt.

Die Darstellungen der Zeichnungen zeigen den erfindungsgemäßen Gegenstand stark schematisiert und sind nicht maßstäblich zu verstehen. Die einzelnen Bestandteile des erfindungsgemäßen Gegenstandes sind so dargestellt, dass ihr Aufbau gut gezeigt werden kann.

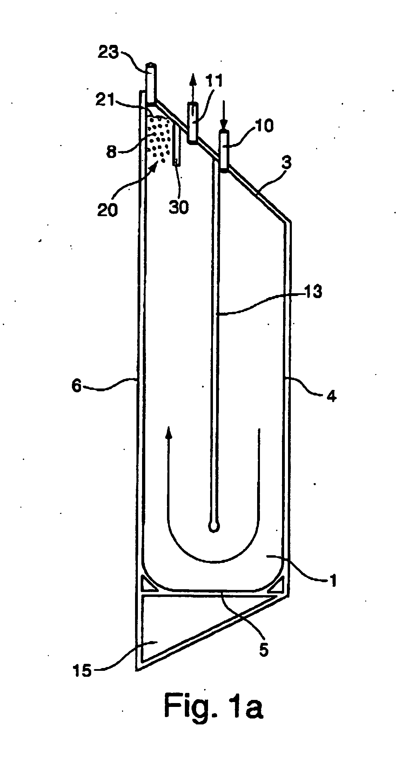

In den Figuren 1a bis 1c sind verschiedene Ausführungsformen eines erfindungsgemäßen Flüssigkeitswärmebeutels dargestellt. Die Flüssigkeitswärmebeutel weisen jeweils ein Durchflussvolumen 1 auf, das aus zwei an vier Begrenzungskanten 3,4,5,6 z.B. durch Verschweißten miteinander verbundenen flexiblen Kunststofffolien gebildet ist. Das Durchflussvolumen 1 hat eine flache, im Wesentlichen trapezförmige Grundform, die durch die Begrenzungskanten 3,4,5,6 begrenzt wird. Die beim Flüssigkeitswärmen oben anzuordnende obere Begrenzungskante 3 verläuft derart schräg zu ihren angrenzenden Begrenzungskanten 4,6, dass im Bereich eines, von der oberen Begrenzungskante 3 und einer angrenzenden Begrenzungskante 6 eingeschlossenen, spitzen Winkels vom Durchflussvolumen 1 ein Gasaufnahmevolumen 8 ausgebildet ist.

An der oberen Begrenzungskante 3 sind jeweils eine Einlassleitungsöffnung 10 und eine Auslassleitungsöffnung 11 für die zu wärmende Flüssigkeit angeordnet. In den Figuren ist die Fließrichtung der Flüssigkeit mit Pfeilen symbolisch dargestellt. Durch eine Flüssigkeitsflussführungsnaht 13, die von einer Verbindung der Kunststofffolien im Bereich des Durchflussvolumens 1 gebildet wird, ist die obere Begrenzungskante 3 in einen unteren Einlassleitungsöffnungsbereich und in einen oberen gasaufnahmevolumenseitigen Auslassleitungsöffnungsbereich aufteilt. Es ist so gewährleistet, dass die Flüssigkeit beim Erwärmen immer den gleichen Fließweg durch den Flüssigkeitswärmebeutel nimmt und gleichmäßig erwärmt wird.

Die Grundform der dargestellten Flüssigkeitswarmebeutel ist lang gestreckt ausgebildet, wobei die sich gegenüberliegenden obere Begrenzungskante 3 und untere Begrenzungskante 5 als Schmalseiten ausgebildet sind und die die obere und die untere Begrenzungskanten verbindenden Begrenzungskanten 4,6 zueinander im Wesentlichen parallel verlaufen und als Langseiten ausgebildet sind. Die Flüssigkeitsflussführungsnaht 13 verläuft an der oberen Begrenzungskante 3 beginnend zu den Langseiten im Wesentlichen parallel. An der der oberen Begrenzungskante 3 gegenüberliegenden unteren Begrenzungskante 5 ist eine Einführlasche 15 ausgebildet. Diese Einführlasche 15 ist als Teil der Kunststofffolien ausgebildet, der über die untere Begrenzungskante 5 herausragt.

Sich beim Erwärmen ausbildende Gasblasen 20 sammeln sich im Gasaufnahmevolumen 8 an. Im Flüssigkeitswärmebeutel aufzuwärmende Flüssigkeit bildet daher einen Flüssigkeitsspiegel 21, d.h. eine Flüssigkeitsoberfläche, im Gasaufnahmevolumen 8 aus. An der oberen Begrenzungskante 3 ist am Bereich des Gasaufnahmevolumens 8 zwischen der Auslassleitungsöffnung 11 und der an den Auslassleitungsöffnungsbereich angrenzenden Begrenzungskante 6 eine Entgasungsöffnung 23 angeordnet, durch die das Gas der Gasblasen 20 aus dem Flüssigkeitswärmebeutel abgeleitet werden kann. Die Entgasungsöffnung ist 23 also weiter oben als die Auslassleitungsöffnung 11 für die erwärmte Flüssigkeit, bevorzugt in der obersten Ecke des Gasaufnahmevolumens 8, angeordnet, so dass aufsteigende Gasblasen 20, und dadurch evtl. ausgebildeter Schaum, sich oberhalb der Auslassleitungsöffnung 11 ansammeln.

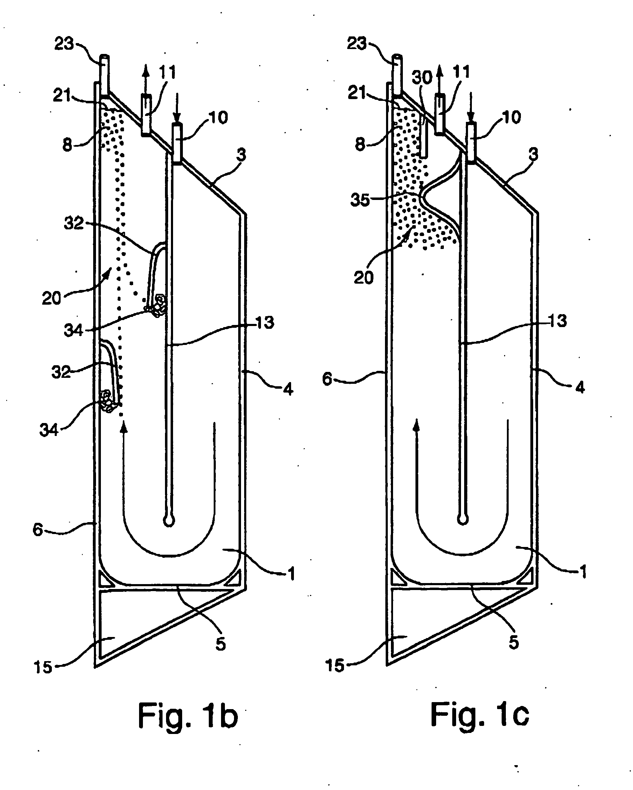

Bei den in den Figuren 1a und 1c dargestellten Ausführungsformen des erfindungsgemäßen Flüssigkeitswärmebeutels ist jeweils eine zwischen der Auslassleitungsöffnung 11 und der Entgasungsöffnung 23 von der oberen Begrenzungskante 3 abgehende und in das Durchflussvolumen 1 hineinragende Trennnaht 30 vorgesehen, die das Gasaufnahmevolumen 8 vom Durchflussvolumen 1 teilweise abtrennt. Die Trennnaht 30 kann z.B. als Verschweißungsnaht der den Flüssigkeitswärmebeutel ausbildenden Kunststofffolien ausgebildet sein.

Bei der in Figur 1b dargestellten Ausführungsform des erfindungsgemäßen Flüssigkeitswärmebeutels sind im auslassleitungsöffnungsseitigen Volumen des Durchflussvolumens 1 zwei Gasblasenaufstiegsbehinderungskanten 32 vorgesehen, die jeweils ein Gassammelvolumen ausbilden. Die Gasblasenaufstiegsbehinderungskanten 32 bilden eine nach unten, also Richtung untere Begrenzungskante 5, offene Volumenabgrenzung aus. Sie ragen jeweils so weit in das Durchflussvolumen 1 hinein, dass über das freie Ende der Gasblasenaufstiegsbehinderungskanten 32 übertließende Gasblasen 20, die z.B. von in den Gassammelvolumina sich ansammelndem Schaum 34 stammen, zum Gasaufnahmevolumen 8 geleitet werden. Auch die Gasblasenaufstiegsbehinderungskanten 32 können z.B. als Verschweißungsnähte der den Flüssigkeitswärmebeutel ausbildenden Kunststofffolien ausgebildet sein.

Bei der in Figur 1c dargestellten Ausführungsform des erfindungsgemäßen Flüssigkeitswärmebeutels ist zusätzlich zu der Trennnaht 30 im ausiassleitungsöffnungsseitigen Volumen des Durchflussvolumens eine Gasblasenführungskante 35 an der Flüssigkeitsflussführungsnaht 13 ausgebildet. Die Gasblasenführungskante 35 ragt ausgehend von der Flüssigkeitsflussführungsnaht 13 in das auslassieltungsöffnungsseitige Volumen des Durchflussvolumens 1 so weit hinein, wie das freie Ende der Trennnaht 30 von der Flüssigkeitsflussführungsnaht 13 beabstandet ist. Auch die Gasblasenführungskante 13 kann z.B, als Verschweißungsnaht der den Flüssigkeitswärmebeutel ausbildenden Kunststofffolien ausgebildet sein. Gasblasen aus der zu erwärmenden Flüssigkeit werden so von der Auslassleitungsöffnung 11 weg geführt um sich im Gasaufnahmevolumen 8 anzusammeln.

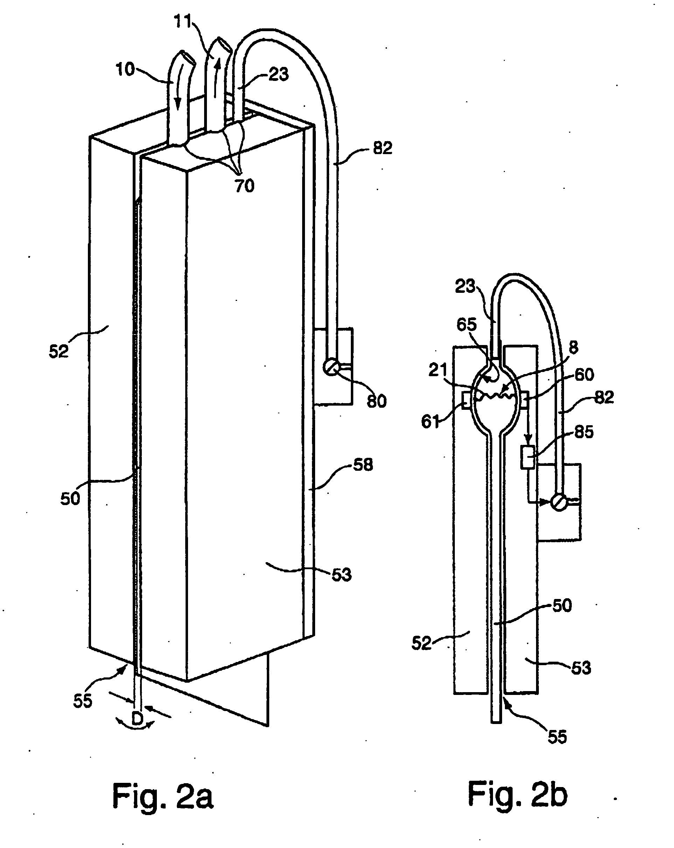

In den Figuren 2a und 2b ist ein erfindungsgemäßer Beutelwärmer mit einem eingeführten erfindungsgemäßen Flüssigkeitswärmebeutel 50 dargestellt, wobei Figur 2b einen schematischen Querschnitt durch den Beutelwärmer mit einem eingeführten Flüssigkeitswärmebeutel 50 zeigt. Zum Aufwärmen der im Flüssigkeitswärmebeutel 50 aufzuwärmenden Flüssigkeit weist der Beutelwärmer zwei im Wesentlichen rechteckige Wärmetauscherplatten 52,53 als Heizelemente auf. Die Wärmetauscherplatten 52,53 bilden im Inneren des Beutelwärmers jeweils eine im Wesentlichen flächige Wärmeaustauschoberfläche aus. Die Wärmetauscherplatten 52,53 sind mit ihren Wärmeaustauschoberflächen derart beabstandet sich gegenüberliegend angeordnet, dass zwischen den Wärmeaustauschoberflächen ein Spalt 55 mit einer Spaltbreite D von einigen Millimetern zur Aufnahme des Flüssigkeitswärmebeutels 50 ausgebildet ist.

Die Wärmetauscherplatten 52,53 sind an einer ersten Längsseitenkante der Wärmetauscherplatten 52,53 mit Befestigungsmitteln 58 derart miteinander befestigt, dass die Wärmeaustauschoberflächen durch die Befestigungsmittel 58 im Wesentlichen parallel zueinander fixiert sind. Dabei ist der Spalt 55 entlang einer zweiten Längsseitenkante und den Schmalseitenkanten der Wärmetauscherplatten offen, wodurch das Einlegen des Flüssigkeitswärmebeutels 50 zwischen die Wärmetauscherplatten 52,53 einfach möglich ist. Die Befestigungsmittel 58 sind derart federnd ausgebildet, dass eine in dem in den Beutelwärmer eingeführten Flüssigkeitswärmebeutel 50 auftretende Druckveränderung zu einer Veränderung der Spaltbreite D führt. Dabei führt eine Druckerhöhung zu einer Aufweitung und eine Druckverminderung zu einer Einengung des Spaltes 55, was durch einen gebogenen Doppelpfeil in der Figur 2a symbolisch dargestellt ist.

Im Bereich der Wärmeaustauschoberflächen ist ein Flüssigkeitsstandsensor 60 zur Detektion eines Flüssigkeitsstandes 21 in dem in den Beutelwärmer eingeführten Flüssigkeitswärmebeutel 50 vorgesehen. Der Flüssigkeitsstandsensor 60 kann z.B. als ein Lichtdetektor in der Wärmeaustauschoberfläche einer der Wärmetauscherplatten 52,53 ausgebildet sein, wobei im auf dem Flüssigkeitsstandsensor 60 gegenüberliegenden Bereich der Wärmeaustauschoberfläche der anderen Wärmetauscherplatte 52 eine Uchtquelle 61 z.B. eine Leuchtdiode positioniert sein kann. Von der Lichtquelle 61 emittiertes Ucht wird dabei je nach Flüssigkeitsstand mehr oder weniger gedämpft vom Flüssigkeitsstandsensor 60 detektiert. Im an die obere Schmalseitenkante der Wärmetauscherplatte 52,53 angrenzenden Bereich der Wärmetauscherplatten 52,53 ist eine Kavität 65 zur Formgebung des Gasaufnahmevolumens 8 des Flüssigkeitswärmebeutels 50 ausgebildet. Der Flüssigkeitswärmebeutel 50 ist also so zwischen den Wärmetauscherplatten 52,53 derart positioniert, dass das Gasaufnahmevolumen 8 des Flüssigkeitswärmebeutels 50 im Bereich der Kavität 65 angeordnet ist. Auch der Flüssigkeitsstandsensor 60 ist im Bereich dieser Kavität 65 positioniert, so dass der Flüssigkeitsstand 21 im Flüssigkeitswärmebeutel 50 im Bereich des Gasaufnahmevolumens 8 detektiert werden kann.

Der Flüssigkeitssensor 60 kann auch eine andere Ausgestaltung haben und ist beispielsweise ergänz- oder ersetzbar durch einen Ultraschallsensor oder durch eine andere Gasblasenerkennungstechnik.

Der Beutelwärmer weist an den oberen Schmalseitenkante der Wärmetauscherplatten 52,53 drei Nutansätze 70 auf, die zu der Einiassleitungsöffnung 10 und zu der Auslassleitungsöffnung 11 und zu der Entgasungsöffnung 23 des in den Beutelwärmier eingeführten Flüssigkeitswärmebeutels 50 derart komplementär ausgeformt sind, dass eine an den Flüssigkeitswärmebeutel 50 angeschlossene Einlaufleitung und eine an den Flüssigkeitswärmebeutel 50 angeschlossene Auslassleitung und eine an den Flüssigkeitswärmebeutel 50 angeschlossene Entgasungsleitung, während der Flüssigkeitswärmebeutel 50 in den Beutelwärmer eingeführt ist, in den Nutansätzen 70 formschlüssig gehalten sind. Am Beutelwärmer ist ein Entgasungsventil 80 zum Anschluss der Entgasungsöffnung 23 des in den Beutelwärmer eingeführten Flüssigkeitswärmebeutels 50 über eine Schlauchleitung 82 vorgesehen. Mittels des Entgasungsventils 80 kann also die Entgasungsöffnung 23 variabel geöffnet oder verschlossen werden. Der Flüssigkeitsstandsensor 60 und das Entgasungsventil 80 sind an eine Steuereinheit 85 angeschlossen. Die Steuereinheit 85 ist eingerichtet, bei einer durch den Flüssigkeitsstandsensor 60 detektierten Überschreitung eines vorbestimmten Flüssigkeltsstarldes 21 im Gasaufnahmevolumen 8 des Flüssigkeitswärmebeutels 50 das Entgasungsventli 80 zum Verschluss der Entgasungsöffnung 23 zu steuern und bei einer durch den Flüssigkeitsstandsensor 60 detektierten Unterschreitung des vorbestimmten Flüssigkeitsstandes 21 im Gasaufnahmevoiumen 8 des Flüssigkeitswärmebeutels 50 das Entgasungsventil 80 zum Öffnen der Entgasungsöffnung 23 zu steuern. Der dabei auftretende Signalfluss ist in Figur 2b durch die Pfeilspitzen der Verbindungen zwischen Flüssigkeitsstandsensor 60 und Steuereinheit 85 sowie zwischen Steuereinheit 85 und Entgasungsventil 80 symbolisiert.

Vorgeschlagen wird ein Flüssigkeitswärmebeutel 50, insbesondere ein Blutwärmebeutel, mit einem, aus mindestens zwei an Begrenzungskanten 3,4,5,6 miteinander verbundenen flexiblen Kunststofffolien gebildeten Durchflussvolumen 1, das eine flache, im Wesentlichen trapezförmige, durch die Begrenzungskanten 3,4,5,6 begrenzte Grundform aufweist, und einer Einlassleitungsöffnung 10 und einer Auslassleitungsöffnung 11 für eine zu wärmende Flüssigkeit, die an einer beim Flüssigkeitswärmen oben anzuordnenden oberen Begrenzungskante 3 der Begrenzungskanten 3,4,5,6 angeordnet sind, wobei die obere Begrenzungskante 3 derart schräg zu ihren angrenzenden Begrenzungskanten 4,6 verläuft, dass im Bereich eines von der oberen Begrenzungskante 3 und einer angrenzenden Begrenzungskante 6 eingeschlossenen spitzen Winkels vom Durchflussvolumen 1 ein Gasaufnahmevolumen 8 ausgebildet ist, sowie einer Elüssigkeitsflussführungsnaht 13, die von einer Verbindung der Kunststofffolien im Bereich des Durchflussvolumens 1 gebildet wird und die obere Begrenzungskante 3 In einen unteren Einlassleitungsöffnungsbereich und in einen oberen gasaufnahmevolumenseitigen Auslassleitungsöffnungsbereich aufteilt. Dabei ist an der oberen Begrenzungskante 3 am Bereich des Gasaufnahmevolumens 8 zwischen der Auslassleitungsöffnung 11 und der an den Auslassleitungsöffnungsbereich angrenzenden Begrenzungskante 6 eine Entgasungsöffnung 23 angeordnet.

Die Erfindung beschränkt sich nicht auf die vorstehend angegebenen Ausführungsbeispiele. Vielmehr ist eine Anzahl von Varianten denkbar, welche auch bei grundsätzlich anders gearteter Ausführung von den Merkmalen der Erfindung Gebrauch machen.

## Patentansprüche

1. Flüssigkeitswärmebeutel (50), insbesondere Blutwärmebeutel, mit
- einem, aus mindestens zwei an Begrenzungskanten (3,4,5,6) miteinander verbundenen flexiblen Kunststofffolien gebildeten Durchflussvolumen (1), das eine flache, im Wesentlichen trapezförmige, durch die Begrenzungskanten (3,4,5,6) begrenzte Grundform aufweist,
- einer Einlassleitungsöffnung (10) und einer Auslassleitungsöffnung (11) für eine zu wärmende Flüssigkeit, die an einer beim Flüssigkeitswärmen oben anzuordnenden oberen Begrenzungskante (3) der Begrenzungskanten (3,4,5,6) angeordnet sind, wobei die obere Begrenzungskante (3) derart schräg zu ihren angrenzenden Begrenzungskanten (4,6) verläuft, dass im Bereich eines von der oberen Begrenzungskante (3) und einer angrenzenden Begrenzungskante (6) eingeschlossenen spitzen Winkels vom Durchflussvolumen (1) ein Gasaufnahmevolumen (8) ausgebildet ist,
- und einer Flüssigkeitsflussführungsnaht (13), die von einer Verbindung der Kunststofffolien im Bereich des Durchflussvolumens (1) gebildet wird und die obere Begrenzungskante (3) in einen unteren Einlassleitungsöffnungsbereich und in einen oberen gasaufnahmevolumenseitigen Auslassleitungsöffnungsbereich aufteilt, **dadurch gekennzeichnet, dass**
an der oberen Begrenzungskante (3) am Bereich des Gasaufnahmevolumens (8) zwischen der Auslassleitungsöffnung (11) und der an den Auslassleitungsöffnungsbereich angrenzenden Begrenzungskante (6) eine Entgasungsöffnung (23) angeordnet ist, wobei
zwischen der Auslassleitungsöffnung (11) und der Entgasungsöffnung (23) eine von der oberen Begrenzungskante (3) abgehende und in das Durchflussvolumen (1) hineinragende Trennnaht (30) vorgesehen ist, die das Gasaufnahmevolumen (8) vom Durchflussvolumen (1) teilweise abtrennt, und wobei
an der Flüssigkeitsflussführungsnaht (13) eine Gasblasenführungskante (35) ausgebildet ist, die ausgehend von der Flüssigkeitsflussführungsnaht (13) in das ausiassleitungsöffnungsseitige Volumen des Durchflussvolumens (1) mindestens so weit hineinragt, wie das freie Ende der Trennnaht (30) von der Flüssigkeitsflussführungsnaht (13) beabstandet ist.

2. Flüssigkeitswärmebeutel nach Anspruch 1,
**dadurch gekennzeichnet, dass**
im auslassleitungsöffnungsseitigen Volumen des Durchflussvolumens (1) mindestens eine Gasblasenaufstiegsbehinderungskante (32) vorgesehen ist, die ein Gassammelvolumen ausbildet.

3. Flüssigkeitswärmebeutel nach einem der Ansprüche 1 bis 2,
**dadurch gekennzeichnet, dass**
eine Einführlasche (15) an einer der oberen Begrenzungskante (3) gegenüberliegenden unteren Begrenzungskante (5) angeordnet ist, und
die Einführlasche (15) als Teil der Kunststofffolien ausgebildet ist, der über die untere Begrenzungskante (5) herausragt.

4. Flüssigkeitswärmebeutel nach einem der Ansprüche 1 bis 3,
**dadurch gekennzeichnet, dass**
das Durchflussvolumen (1) dadurch ausgebildet ist, dass die Kunststofffolien an den Begrenzungskanten (3,4,5,6) direkt miteinander verschweißt sind und die Flüssigkeitsflussführungsnaht (13) als eine Schweißnaht zwischen den Kunststofffolien ausgebildet ist.

5. Flüssigkeitswärmebeutel nach einem der Ansprüche 1 bis 4,
**dadurch gekennzeichnet, dass**
die Grundform langgestreckt ausgebildet ist, wobei die sich gegenüberliegenden obere (3) und untere Begrenzungskanten (5) als Schmalseiten ausgebildet sind und die die obere und die untere Begrenzungskanten (3,5) verbindenden Begrenzungskanten (4,6) zueinander im Wesentlichen Parallel verlaufen und als Langseiten ausgebildet sind, wobei die Flüssigkeitsflussführungsnaht (13) beginnend an der oberen Begrenzungskante (3) zu den Langseiten im Wesentlichen parallel verläuft.

6. Flüssigkeitswärmesystem mit einem Flüssigkeitswärmebeutel (50) nach mindestens einem der Ansprüche 1 bis 5 und einem Beutelwärmer zum Einführen des Flüssigkeitswärmebeutels (50) mit Heizelementen, die zwei im Wesentlichen rechteckige Wärmetauscherplatten (52,53) mit jeweils einer im Wesentlichen flächigen Wärmeaustauschoberfläche ausbilden,
- wobei die Wärmetauscherplatten (52,53) mit deren Wärmeaustauschoberfläche derart beabstandet sich gegenüberliegend angeordnet sind, dass zwischen den Wärmeaustauschoberflächen ein Spalt (55) mit einer Spaltbreite D zur Aufnahme eines Flüssigkeitswärmebeutels (50) ausgebildet ist, **dadurch gekennzeichnet, dass** im Bereich der Wärmeaustauschoberflächen mindestens ein Flüssigkeitsstandsensor (60) und/oder eine Beobachtungsöffnung zur Detektion eines Flüssigkeitsstandes (21) in dem in den Beutelwärmer eingeführten Flüssigkeitswärmebeutel (50) vorgesehen ist.

7. Flüssigkeitswärmesystem nach Anspruch 6
**dadurch gekennzeichnet, dass**
der Beutelwärmer an einer oberen Schmalseitenkante der Wärmetauscherplatten (52,53) drei Nutansätze (70) aufweist, wobei die Nutansätze (70) zu der Einlassleitungsöffnung (10) und zu der Auslassleitungsöffnung (11) und zu der Entgasungsöffnung (23) des in den Beutelwärmer eingeführten Flüssigkeitswärmebeutels (50) derart komplementär ausgeformt sind, dass eine an den Flüssigkeitswärmebeutel (50) angeschlossene Einlaufleitung und eine an den Flüssigkeitswärmebeutel (50) angeschlossene Auslassleitung und eine an den Flüssigkeitswärmebeutel (50) angeschlossene Entgasungsleitung, während der Flüssigkeitswärmebeutel (50) in den Beutelwärmer eingeführt ist, in den Nutansätzen (70) formschlüssig gehalten sind.

8. Flüssigkeitswärmesystem nach Anspruch 7,
**dadurch gekennzeichnet, dass**
in mindestens einer der Wärmetauscherplatten (52,53) im an die obere Schmalseitenkante der Wärmetauscherplatte (52,53) angrenzenden Bereich eine Kavität (65), insbesondere eine Einfräsung, zur Formgebung des Gasaufnahmevolumens (8) des Flüssigkeitswärmebeutels (50) ausgebildet ist.

9. Flüssigkeitswärmesystem nach einem der Ansprüche 7 bis 8,
**dadurch gekennzeichnet, dass**
am Beutelwärmer ein Entgasungsventil (80) zum Anschluss der Entgasungsöffnung (23) des in den Beutelwärmer eingeführten Flüssigkeitswärmebeutels (50), bevorzugt über eine Schlauchleitung (82), vorgesehen ist.

10. Flüssigkeitswärmesystem nach Anspruch 9,
**dadurch gekennzeichnet, dass**
der Flüssigkeitsstandsensor (60) und das Entgasungsventil (80) an eine Steuereinheit (85) angeschlossen sind, wobei die Steuereinheit (85) eingerichtet ist, bei einer durch den Flüssigkeitsstandsensor (60) detektierten Überschreitung eines vorbestimmten Flüssigkeitsstandes (21) im Gasaufnahmevolumen (8) des Flüssigkeitswärmebeutels (50) das Entgasungsventil (80) zum Verschluss der Entgasungsöffnung (23) zu steuern und
bei einer durch den Flüssigkeitsstandsensor (60) detektierten Unterschreitung des vorbestimmten Flüssigkeitsstandes (21) im Gasaufnahmevolumen (8) des Flüssigkeitswärmebeutels (50) das Entgasungsventil (80) zum Öffnen der Entgasungsöffnung (23) zu steuern und/oder ein Abpumpen von im Gasaufnahmevolumen (8) vorhandenem Gas mittels einer Pumpe zu steuern.

11. Flüssigkeitswärmesystem nach einem der Ansprüche 6 bis 10, **dadurch gekennzeichnet, dass**
die Wärmetauscherplatten (52,53) an einer ersten Längsseitenkante der Wärmetauscherplatten (52,53) mit Befestigungsmitteln (58) derart miteinander befestigt sind, dass die Wärmeaustauschoberflächen durch die Befestigungsmittel (58) im Wesentlichen parallel zueinander fixiert sind,
wobei der Spalt (55) entlang einer zweiten Längsseitenkante und den Schmalseitenkanten der Wärmetauscherplatten (52,53) offen ist, wobei
die Befestigungsmittel (58) derart federnd ausgebildet sind, dass eine in einem in den Beutelwärmer eingeführten Flüssigkeitswärmebeutel (50) auftretende Druckveränderung zu einer Veränderung der Spaltbreite D führt, wobei eine Druckerhöhung zu einer Aufweitung und eine Druckverminderung zu einer Einengung des Spaltes (55) führt.

## Claims

1. Liquid heating bag (50), in particular, a blood heating bag, with
- a flow volume (1) formed from at least two flexible plastic foils connected to each other at delimiting edges (3,4,5,6) and having a flat, substantially trapezoidal basic shape which is delimited by the delimiting edges (3,4,5,6),
- an inlet line opening (10) and an outlet line opening (11) for liquid to be heated, which are disposed on an upper delimiting edge (3) of the delimiting edges (3,4,5,6), which is to be disposed at the top during liquid heating, wherein the upper delimiting edge (3) extends sufficiently obliquely with respect to its adjacent delimiting edges (4,6) such that a gas reception volume (8) is formed in the region of an acute angle of the flow volume (1) subtended between the upper delimiting edge (3) and a delimiting edge (6) adjacent thereto,
- and a liquid flow guiding seam (13) that is formed by connecting the plastic foils in the region of the flow volume (1) and that divides the upper delimiting edge (3) into a lower inlet line opening region and an upper outlet line opening region on the gas reception volume side, **characterized in that**
a degassing opening (23) is disposed at the upper delimiting edge (3) in the region of the gas reception volume (8) between the outlet line opening (11) and the delimiting edge (6) adjacent to the outlet line opening region,
wherein a separating seam (30) is provided between the outlet line opening (11) and the degassing opening (23), which extends from the upper delimiting edge (3) to protrude into the flow volume (1) and partially separates the gas reception volume (8) from the flow volume (1), and wherein
a gas bubble guiding edge (35) is formed at the liquid flow guiding seam (13) which, extending from the liquid flow guiding seam (13), protrudes into the volume of the flow volume (1) on the side of the outlet line opening at least as far as the free end of the separating seam (30) is separated from the liquid flow guiding seam (13).

2. Liquid heating bag according to claim 1,
**characterized in that**,
in the volume of the flow volume (1) on the outlet line opening side, at least one gas bubble rise impediment edge (32) is provided that forms a gas collection volume.

3. Liquid heating bag according to any one of the claims 1 to 2, **characterized in that**
an insertion tab (15) is disposed on a lower delimiting edge (5) opposite the upper delimiting edge (3),
and
the insertion tab (15) is formed as a part of the plastic foils that projects past the lower delimiting edge (5).

4. Liquid heating bag according to any one of the claims 1 to 3, **characterized in that**
the flow volume (1) is formed by directly welding the plastic foils to each other at the delimiting edges (3,4,5,6) and the liquid flow guiding seam (13) is formed as a weld seam between the plastic foils.

5. Liquid heating bag according to any one of the claims 1 to 4, **characterized in that**
the basic shape is elongated, wherein the upper (3) and lower delimiting edges (5) located opposite each other are formed as narrow sides and the delimiting edges (4,6) connecting the upper and the lower delimiting edges (3,5) essentially extend parallel to each other and are formed as long sides, wherein the liquid flow guiding seam (13) extends from the upper delimiting edge (3) essentially parallel to the long sides.

6. Liquid heating system with a liquid heating bag (50) according to at least one of the claims 1 to 5, and a bag heating means for introducing the liquid heating bag (50) with heating elements that form two substantially rectangular heat exchanger plates (52,53) each having a substantially flat heat exchanging surface,
- wherein the heat exchanger plates (52,53) are disposed opposite each other with their heat exchanging surfaces having a distance from each other such that a gap (55) is formed between the heat exchanging surfaces, with a gap width D for receiving the liquid heating bag (50),
**characterized in that**,
in the region of the heat exchanger surfaces, at least one liquid level sensor (60) and/or one monitoring opening are provided for detecting a liquid level (21) in the liquid heating bag (50) which is inserted into the bag heating means.

7. Liquid heating system according to claim 6,
**characterized in that**
the bag heating means has three grooved shoulders (70) at an upper narrow side edge of the heat exchanger plates (52,53), wherein the grooved shoulders (70) are shaped complementarily to the inlet line opening (10), to the outlet line opening (11) and to the degassing opening (23) of the liquid heating bag (50) inserted into the bag heating means such that an inlet line connected to the liquid heating bag (50), an outlet line connected to the liquid heating bag (50), and a degassing line connected to the liquid heating bag (50) are
positively held in the grooved shoulders (70) while the liquid heating bag (50) is inserted into the bag heating means.

8. Liquid heating system according to claim 7,
**characterized in that,**
in at least one of the heat exchanger plates (52,53) in the region adjacent to the upper narrow side edge of the heat exchanger plate (52,53), a cavity (65), in particular, a cut recess is formed for shaping the gas reception volume (8) of the liquid heating bag (50).

9. Liquid heating system according to any one of the claims 7 to 8,
**characterized in that**
a degassing valve (80) is provided on the bag heating means for connection, preferably via a tube (82), of the degassing opening (23) of the liquid heating bag (50) which is inserted into the bag heating means.

10. Liquid heating system according to claim 9,
**characterized in that**
the liquid level sensor (60) and the degassing valve (80) are connected to a control unit (85), wherein the control unit (85) is set up in such a way as to control the degassing valve (80) to close the degassing opening (23) when the liquid level (21) detected by the liquid level sensor (60) rises above a predefined liquid level in the gas reception volume (8) of the liquid heating bag (50) and to control the degassing valve (80) to open the degassing opening (23) and/or to control pumping away of the gas in the gas reception volume (8) by means of a pump when the liquid level (21) detected by the liquid level sensor (60) falls below the predefined liquid level in the gas reception volume (8) of the liquid heating bag (50).

11. Liquid heating system according to any one of the claims 6 to 10,
**characterized in that**
the heat exchanger plates (52,53) are mounted to each other on a first longitudinal side edge of the heat exchanger plates (52,53) using fastening means (58) such that the heat exchanging surfaces are fixed substantially parallel to each other via the fastening means (58),
wherein the gap (55) is open along a second longitudinal side edge and the narrow side edges of the heat exchanger plates (52,53) wherein
the fastening means (58) are resilient such that a pressure change in a liquid heating bag (50) that is inserted into the bag heating means changes the gap width D, wherein a pressure increase widens the gap and a pressure reduction reduces the size of the gap (55).

## Revendications

1. Récipient (50) de réchauffage de fluides, notamment poche de réchauffage du fluide sanguin, comprenant
- un espace de circulation (1) constitué d'au moins deux films souples en matière plastique reliés l'un à l'autre sur des arêtes de délimitation (3, 4, 5, 6), et doté d'une configuration de base aplatie pour l'essentiel trapézoïdale, délimitée par lesdites arêtes de délimitation (3, 4, 5, 6),
- un orifice (10) dédié à un conduit d'admission et un orifice (11) dédié à un conduit d'évacuation d'un fluide devant être réchauffé, pratiqués sur une arête supérieure de délimitation (3) qui, parmi lesdites arêtes de délimitation (3, 4, 5, 6), est conçue pour être placée en partie haute au cours du réchauffage du fluide, ladite arête supérieure de délimitation (3) s'étendant à l'oblique, vis-à-vis des arêtes de délimitation (4, 6) qui lui sont adjacentes, de manière que ledit espace de circulation (1) donne naissance à un volume (8) de réception de gaz dans la région d'un angle aigu décrit par ladite arête supérieure de délimitation (3) et par une arête de délimitation (6) adjacente,
- et une lamelle (13) de guidage de l'écoulement du fluide, formée par une solidarisation des films de matière plastique dans la région dudit espace de circulation (1), et scindant ladite arête supérieure de délimitation (3) en une zone inférieure dévolue audit orifice dédié au conduit d'admission, et en une zone supérieure située du côté dudit volume de réception de gaz et dévolue audit orifice dédié au conduit d'évacuation, **caractérisé par le fait**
**qu'**un orifice de dégazage (23) est pratiqué sur l'arête supérieure de délimitation (3), dans la région du volume (8) de réception de gaz, entre l'orifice (11) dédié au conduit d'évacuation et l'arête de délimitation (6) adjacente à la zone dévolue audit orifice dédié audit conduit d'évacuation,
sachant
**qu'**une lamelle séparatrice (30) prévue entre ledit orifice de dégazage (23) et ledit orifice (11) dédié au conduit d'évacuation, partant de ladite arête supérieure de délimitation (3) et s'engageant dans l'espace de circulation (1), sépare en partie ledit volume (8) de réception de gaz d'avec ledit espace de circulation (1), et sachant
**qu'**une arête (35) de guidage de bulles gazeuses, ménagée sur la lamelle (13) de guidage de l'écoulement du fluide, part de ladite lamelle (13) de guidage de l'écoulement du fluide et s'engage, dans le volume dudit espace de circulation (1) qui est situé du côté dudit orifice dédié au conduit d'évacuation, au moins d'une distance correspondant à l'espacement entre l'extrémité libre de ladite lamelle séparatrice (30) et ladite lamelle (13) de guidage de l'écoulement du fluide.

2. Récipient de réchauffage de fluides, selon la revendication 1,
**caractérisé par le fait**
**qu'**au moins une arête (32) empêchant une ascension de bulles gazeuses, donnant naissance à un volume collecteur de gaz, est prévue dans le volume de l'espace de circulation (1) qui est situé du côté de l'orifice dédié au conduit d'évacuation.

3. Récipient de réchauffage de fluides, selon l'une des revendications 1 à 2,
**caractérisé par le fait**
**qu'**une patte d'insertion (15) est disposée sur une arête inférieure de délimitation (5) tournée à l'opposé de l'arête supérieure de délimitation (3), et
**que** ladite patte d'insertion (15) se présente comme une partie des films de matière plastique qui fait saillie au-delà de ladite arête inférieure de délimitation (5).

4. Récipient de réchauffage de fluides, selon l'une des revendications 1 à 3,
**caractérisé par le fait**
**que** l'espace de circulation (1) est formé en reliant directement les films de matière plastique l'un à l'autre, par soudage, sur les arêtes de délimitation (3, 4, 5, 6), et la lamelle (13) de guidage de l'écoulement du fluide revêt la forme d'un joint soudé entre lesdits films de matière pastique.

5. Récipient de réchauffage de fluides, selon l'une des revendications 1 à 4,
**caractérisé par le fait**
**que** la configuration de base offre un profil allongé, sachant que les arêtes supérieure (3) et inférieure (5) de délimitation, tournées à l'opposé l'une de l'autre, se présentent comme des petits côtés et que les arêtes de délimitation (4, 6), reliant l'une à l'autre lesdites arêtes supérieure et inférieure de délimitation (3, 5), s'étendent pour l'essentiel parallèlement l'une à l'autre et se présentent comme des grands côtés, la lamelle (13) de guidage de l'écoulement du fluide, débutant sur ladite arête supérieure de délimitation (3), s'étendant alors, pour l'essentiel, parallèlement auxdits grands côtés.

6. Système de réchauffage de fluides, équipé d'un récipient (50) de réchauffage de fluides conforme à au moins l'une des revendications 1 à 5, et d'un chauffe-récipients prévu pour l'insertion dudit récipient (50) de réchauffage de fluides et présentant des éléments chauffants qui matérialisent deux panneaux d'échange thermique (52, 53) sensiblement rectangulaires, respectivement pourvus d'une surface d'échange de chaleur substantiellement plane,
- lesdits panneaux d'échange thermique (52, 53) étant placés en regard l'un de l'autre par leurs surfaces d'échange de chaleur, avec un espacement propre à réserver, entre lesdites surfaces d'échange de chaleur, un interstice (55) de largeur D destiné à recevoir un récipient (50) de réchauffage de fluides,
**caractérisé par le fait**
**qu'**au moins un détecteur (60) de niveaux de fluides, et/ou un orifice d'observation affecté à la détection d'un niveau de fluide (21) dans le récipient (50) de réchauffage de fluides inséré dans le chauffe-récipients, est (sont) prévu(s) dans la région des surfaces d'échange de chaleur.

7. Système de réchauffage de fluides, selon la revendication 6,
**caractérisé par le fait**
**que** le chauffe-récipients comporte trois embouchures échancrées (70), sur une arête supérieure matérialisant un petit côté des panneaux d'échange thermique (52, 53), lesdits embouchures échancrées (70) offrant un profil complémentaire de ceux de l'orifice (10) dédié au conduit d'admission, de l'orifice (11) dédié au conduit d'évacuation et de l'orifice de dégazage (23) du récipient (50) de réchauffage de fluides inséré dans ledit chauffe-récipients, de façon telle qu'un conduit d'admission raccordé audit récipient (50) de réchauffage de fluides, un conduit d'évacuation raccordé audit récipient (50) de réchauffage de fluides, et un conduit de dégazage raccordé audit récipient (50) de réchauffage de fluides, soient retenus dans lesdites embouchures échancrées (70), par complémentarité de formes, au stade de l'insertion dudit récipient (50) de réchauffage de fluides dans ledit chauffe-récipients.

8. Système de réchauffage de fluides, selon la revendication 7,
**caractérisé par le fait**
**qu'**une cavité (65), en particulier une dépouille fraisée, est prévue dans au moins l'un des panneaux d'échange thermique (52, 53) dans la région attenante à l'arête supérieure matérialisant le petit côté dudit panneau d'échange thermique (52, 53), de manière à profiler le volume (8) de réception de gaz du récipient (50) de réchauffage de fluides.

9. Système de réchauffage de fluides, selon l'une des revendications 7 à 8,
**caractérisé par le fait**
**qu'**un clapet de dégazage (80) est prévu sur le chauffe-récipients pour permettre le raccordement, de préférence par l'intermédiaire d'un conduit souple (82), de l'orifice de dégazage (23) du récipient (50) de réchauffage de fluides inséré dans ledit chauffe-récipients.

10. Système de réchauffage de fluides, selon la revendication 9,
**caractérisé par le fait**
**que** le détecteur (60) de niveaux de fluides et le clapet de dégazage (80) sont connectés à une unité de commande (85), ladite unité de commande (85) étant agencée pour piloter ledit clapet de dégazage (80) dans le sens d'une obturation de l'orifice de dégazage (23) en cas de dépassement, détecté par ledit détecteur (60) de niveaux de fluides, d'un niveau de fluide prédéterminé (21) dans le volume (8) de réception de gaz du récipient (50) de réchauffage de fluides ; et pour piloter ledit clapet de dégazage (80) dans le sens d'une ouverture dudit orifice de dégazage (23), et/ou pour piloter une élimination de gaz présents dans ledit volume (8) de réception de gaz, par pompage au moyen d'une pompe, en cas de dépassement négatif, détecté par ledit détecteur (60) de niveaux de fluides, dudit niveau de fluide prédéterminé (21) dans ledit volume (8) de réception de gaz dudit récipient (50) de réchauffage de fluides.

11. Système de réchauffage de fluides, selon l'une des revendications 6 à 10, **caractérisé par le fait**
**que** les panneaux d'échange thermique (52, 53) sont fixés l'un à l'autre par des moyens de fixation (58), sur une première arête matérialisant un grand côté desdits panneaux d'échange thermique (52, 53), de telle sorte que les surfaces d'échange de chaleur soient consignées à demeure pour l'essentiel parallèlement l'une à l'autre, par lesdits moyens de fixation (58),
l'interstice (55) étant ouvert le long d'une seconde arête matérialisant un grand côté, et le long des arêtes matérialisant les petits côtés desdits panneaux d'échange thermique (52, 53),
lesdits moyens de fixation (58) présentant une réalisation élastique telle qu'une variation de pression, survenant dans un récipient (50) de réchauffage de fluides inséré dans le chauffe-récipients, gouverne une variation de la largeur D dudit interstice, sachant qu'un accroissement de pression et une diminution de pression impliquent, respectivement, un élargissement et un rétrécissement dudit interstice (55).
